(19) Europäisches Patentamt
European Patent Office
Office européen des brevets

(11) **EP 4 737 585 A1**

(12) **EUROPEAN PATENT APPLICATION**

(43) Date of publication:
**06.05.2026  Bulletin 2026/19**

(21) Application number: **25212995.2**

(22) Date of filing: **03.11.2025**

(51) International Patent Classification (IPC):
*C12Q 1/26* (2006.01)   *C12Q 1/28* (2006.01)
*G01N 33/542* (2006.01)   *G01N 33/58* (2006.01)

(52) Cooperative Patent Classification (CPC):
**C12Q 1/28; C12Q 1/26; G01N 33/542;
G01N 33/582**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **05.11.2024  JP 2024193754**

(71) Applicant: **Canon Kabushiki Kaisha
Tokyo 146-8501 (JP)**

(72) Inventors:
• **YAMAUCHI, Fumio**
**Tokyo (JP)**
• **KAKEGAWA, Norishige**
**Tokyo (JP)**
• **KITAGAWA, Kenji**
**Tokyo (JP)**
• **NOMOTO, Tsuyoshi**
**Tokyo (JP)**

(74) Representative: **WESER & Kollegen
Patentanwälte PartmbB
Radeckestraße 43
81245 München (DE)**

(54) **METHOD OF DETECTING TARGET SUBSTANCE AND REAGENT FOR INSPECTION OF TARGET SUBSTANCE**

(57)     Provided are a highly sensitive detection method for a target substance and a reagent for inspection thereof. Specifically, provided is a method of detecting a target substance that is a substance related to an enzyme reaction, the method including: mixing a specimen liquid that may contain the target substance and a reagent to obtain a liquid sample containing hydrogen peroxide, a peroxidase (40), a phenol derivative and a plurality of luminescent particles (10) each having a binding functional group; aggregating the plurality of luminescent particles (10) through binding of the binding functional groups (20) of the luminescent particles (10) based on a reaction of the hydrogen peroxide, the peroxidase (40) and the phenol derivative in the liquid sample, which occurs when the target substance is present in the liquid sample; and obtaining a value related to fluorescence anisotropy of the liquid sample.

FIG. 1

FIRST STEP
OF MIXING SPECIMEN LIQUID THAT MAY CONTAIN TARGET SUBSTANCE AND REAGENT TO OBTAIN LIQUID SAMPLE CONTAINING HYDROGEN PEROXIDE, PEROXIDASE, PHENOL DERIVATIVE AND PLURALITY OF LUMINESCENT PARTICLES EACH HAVING BINDING FUNCTIONAL GROUP
— S1001

SECOND STEP
OF AGGREGATING PLURALITY OF LUMINESCENT PARTICLES THROUGH BINDING OF BINDING FUNCTIONAL GROUPS OF LUMINESCENT PARTICLES BASED ON REACTION OF HYDROGEN PEROXIDE, PEROXIDASE AND PHENOL DERIVATIVE IN LIQUID SAMPLE, WHICH OCCURS WHEN TARGET SUBSTANCE IS PRESENT IN LIQUID SAMPLE
— S1002

THIRD STEP
OF OBTAINING VALUE RELATED TO FLUORESCENCE ANISOTROPY OF LIQUID SAMPLE IN SECOND STEP
— S1003

FOURTH STEP
OF DETECTING TARGET SUBSTANCE BASED ON VALUE RELATED TO FLUORESCENCE ANISOTROPY
— S1004

EP 4 737 585 A1

**Description**

TECHNICAL FIELD

The present disclosure relates to a method of detecting a target substance and a reagent for inspection of a target substance.

BACKGROUND

Enzymes have a role of catalyzing chemical reactions in a living body, and measurement of enzyme activity is extremely important in the fields of not only medical research of enzymes but also inspection of enzymes, clinical inspections utilizing enzymes as reagents and substance production utilizing enzymes.

Hitherto, enzyme activity has been measured by various methods. In general, a method using a natural or synthetic substrate for an individual enzyme is performed. In the method using a synthetic substrate, the enzyme activity can be measured by utilizing the enzyme activity and optically detecting and measuring a dye that is liberated from the synthetic substrate by the action of the enzyme. Alternatively, for example, a substrate is used in which luminescence is weak before the action of the enzyme, but the luminescence intensity increases after the action.

However, when the enzyme concentration is extremely low, the amount of a fluorescent dye liberated from the synthetic substrate is also small, and hence the luminescence from the fluorescent dye weakens. Thus, there has been a problem in that the measurement of the enzyme activity becomes difficult, or measurement accuracy decreases. In addition, the fluorescent dye is liable to be affected by the surrounding environment, and for example, in a liquid sample containing a large amount of contaminants such as blood, the dye adsorbs to contaminants, such as proteins and lipids, and the fluorescent properties of the substrate change. As a result, high-sensitivity measurement becomes difficult in some cases. Further, in order to eliminate the influence of contaminants, it is possible to separate the enzyme that is a target substance or the contaminants, but the separation work is complicated, and the measurement time may lengthen.

Fluorescence polarization measurement is known as one of the methods of measuring enzyme activity. The fluorescence polarization measurement, which utilizes fluorescence polarization, is a method of measuring the rotational motion of a fluorescent substance. When the fluorescent substance is excited, the polarization of its fluorescence (also referred to as "degree of fluorescence polarization") changes in accordance with the rotational motion of the fluorescent substance. That is, the method utilizes the fact that when the fluorescent substance does not rotate, polarized luminescence is observed, whereas when the fluorescent substance freely rotates, fluorescence is radiated in all planes, and the polarization is eliminated. A feature of the fluorescence polarization measurement is that in the assay, the degree of fluorescence polarization is used as an indicator, not the luminescence intensity of the fluorescence. Another feature is that the method does not require a separation or washing operation. That is, a homogeneous assay is possible, and a specimen can be added to a solution and measured as it is. Thus, complicated separation work is unnecessary, and measurement can be performed in a short period of time.

Methods of measuring enzyme activity by the fluorescence polarization measurement have heretofore been disclosed. In Japanese Patent Laid-Open No. H10-099097, there is a disclosure of the measurement of the activity of a protease, which is a protein-degrading enzyme. The measurement utilizes the fact that when a substrate molecule having a luminescent substance is cleaved by the protease, the size of the luminescent substance decreases, and the mobility of the luminescent substance changes. In U.S. Patent Application Publication No. 2008/0145880, there is a disclosure of the measurement of the activity of an enzyme that catalyzes a phosphoric acid modification, including a kinase, a phosphatase, a cyclase and a phosphodiesterase.

In U.S. Patent Application Publication No. 2024/0093087, there is a disclosure of an immunoassay technology using fluorescence polarization measurement. In U.S. Patent Application Publication No. 2024/0093087, a high-sensitivity immunoassay is constructed by using luminescent particles to aggregate the luminescent particles via an antigen-antibody reaction, and by observing a change in degree of fluorescence polarization before and after the aggregation.

However, it has not been possible to measure a substance related to an enzyme reaction of a peroxidase in a liquid sample in a simple manner and with high sensitivity.

SUMMARY

As a result of intensive investigations to solve the above-mentioned problems, the inventors have found that fluorescence polarization measurement of the activity or amount of an enzyme or the amount of an enzyme reaction-related substance can be performed in a short period of time and with high sensitivity by introducing a mechanism that aggregates a plurality of luminescent particles by the action of an oxidoreductase in a liquid sample to greatly reduce the mobility of the luminescent particles. Thus, the inventors have completed the present invention. That is, the present disclosure provides a method of measuring the mobility of luminescent particles in a liquid sample using a value related to fluorescence anisotropy.

The present disclosure in its first aspect provides a method of detecting a target substance that is a substance related to an enzyme reaction, the method including: a first step of mixing a specimen liquid that may contain the target substance and a reagent to obtain a liquid sample containing hydrogen peroxide, a peroxidase, a phenol derivative and a plurality of luminescent particles each having a binding functional group; a second step of aggregating the plurality of luminescent particles through binding of the binding functional groups of the luminescent particles based on a reaction of the hydrogen peroxide, the peroxidase and the phenol derivative in the liquid sample, which occurs when the target substance is present in the liquid sample; and a third step of obtaining a value related to fluorescence anisotropy of the liquid sample in the second step as specified in claim 1. Optional features are specified in claims 2 to 10.

Further, the present disclosure in its second aspect provides a reagent for inspection of a target substance using measurement of a value related to fluorescence anisotropy, wherein the target substance is a peroxidase, and the reagent contains hydrogen peroxide, a phenol derivative and a plurality of luminescent particles each having a binding functional group; wherein the target substance is a hydrogen peroxide, and the reagent contains a peroxidase, a phenol derivative and a plurality of luminescent particles each having a binding functional group; or wherein the target substance is a phenol derivative, and the reagent contains a peroxidase, hydrogen peroxide and a plurality of luminescent particles each having a binding functional group as specified in claim 11. Optional features are specified in claims 13 and 14.

Further, the present disclosure in its third aspect provides a reagent for inspection of a target substance using measurement of a value related to fluorescence anisotropy, wherein the target substance is an oxidase, and the reagent contains a substrate for the oxidase, a peroxidase, hydrogen peroxide, a phenol derivative and a plurality of luminescent particles each having a binding functional group; or wherein the target substance is a substrate for an oxidase, and the reagent contains the oxidase, a peroxidase, hydrogen peroxide, a phenol derivative and a plurality of luminescent particles each having a binding functional group as specified in claim 12. Optional features are specified in claims 13 and 14.

According to the present disclosure, a substance related to an enzyme reaction can be measured with high sensitivity and rapidly, for example, even when the amount of an enzyme is small or the enzyme activity is low.

Features of the present disclosure will become apparent from the following description of embodiments with reference to the attached drawings. The following description of embodiments is described by way of example.

BRIEF DESCRIPTION OF THE DRAWINGS

Fig. 1 is a diagram for illustrating the steps of a method of detecting a target substance according to one aspect of the present disclosure.
Fig. 2 is a diagram for illustrating a highly sensitive measurement method for a peroxidase according to an embodiment of the present disclosure.
Fig. 3 is a diagram for illustrating a measurement method for an oxidase according to an embodiment of the present disclosure.
Fig. 4 is a diagram for illustrating a measurement method for a hydrogen peroxide concentration according to an embodiment of the present disclosure.
Fig. 5 is a diagram for illustrating a measurement method for a glucose concentration according to an embodiment of the present disclosure.

DESCRIPTION OF THE EMBODIMENTS

The present disclosure is described in more detail below.

In the present disclosure, the term "detection" of a target substance refers to the determination of not only the presence or absence of the target substance but also the content, concentration or activity thereof. The term is used in a sense including both qualitative and quantitative cases, and may also be referred to as "measurement".

(Degree of Fluorescence Polarization determined by Fluorescence Polarization Measurement)

Fluorescence polarization measurement is used to analyze the mobility of fluorescent molecules in a solution. The principle of the fluorescence polarization measurement is described by taking the case of luminescent particles of the present disclosure as an example. When luminescent particles (the particles include luminescent molecules exhibiting fluorescence polarization) in a liquid are excited by plane-polarized light, the particles emit polarized fluorescence in the same plane. However, when the luminescent particles rotate by Brownian motion during the excited state, the particles emit fluorescence to a plane different from the excitation plane, and thus their degree of fluorescence polarization is eliminated. In other words, the degree of fluorescence polarization represents the degree of rotational motion of the luminescent particles from being excited to emitting fluorescence.

A method of detecting a target substance according to an embodiment of the present disclosure is a method that utilizes measurement of the mobility of luminescent particles in a liquid sample. When the luminescent particles in a liquid are dispersed singly in a solution, the particles rotate vigorously by their Brownian motion, and thus exhibit a low degree of fluorescence polarization. Meanwhile, when the luminescent particles aggregate and the apparent size of the luminescent particles increases, the Brownian motion in the solution decreases, and the degree of fluorescence polarization increases. Accordingly, in the fluorescence polarization measurement, the mobility of the luminescent particles in the solution is analyzed by using a change in degree of fluorescence polarization as an indicator. The degree of fluorescence polarization may be indicated by milli P (hereinafter abbreviated as "mp"), which represents a change in plane polarization.

The change in mobility of the luminescent particles may be detected as a change in degree of fluorescence polarization, that is, may be utilized in the detection of various target substances in a liquid sample by controlling the mobility.

In this embodiment, it has been described that the evaluation of the mobility of the luminescent particles may be evaluated by using the change in degree of fluorescence polarization as an indicator. In the present disclosure, however, the evaluation of the mobility of the luminescent particles only needs to be performed by using a change in value related to fluorescence anisotropy as an indicator, and polarization anisotropy may be used.

(Method of detecting Target Substance)

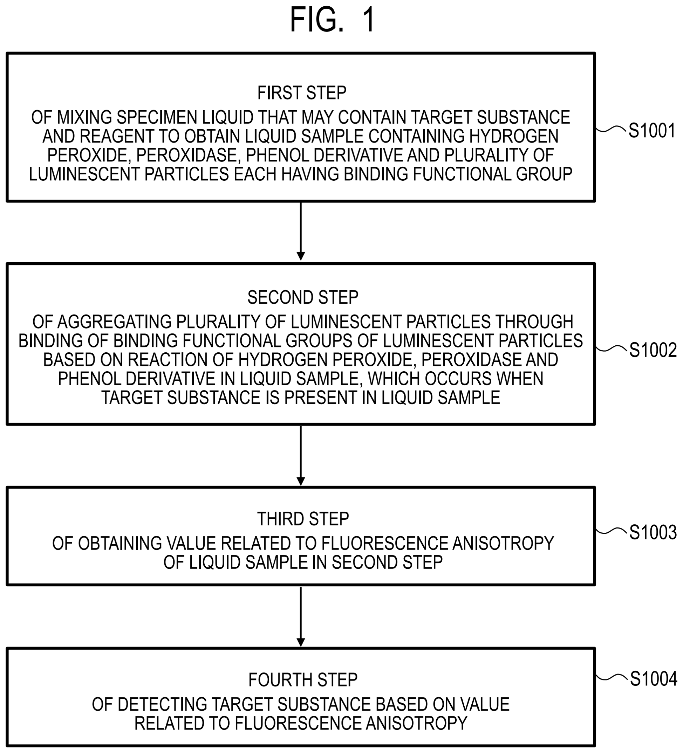

The method of detecting a target substance of this embodiment is a method of detecting a target substance including a substance related to an enzyme reaction, the method including the following steps (1) to (3). Here, at the time of the detection of the target substance, the method may include a step (4) of detecting the target substance using a value related to fluorescence anisotropy obtained in the step (3), as illustrated in Fig. 1.

(1) A first step (S1001) of mixing a specimen liquid that may contain the target substance and a reagent to obtain a liquid sample containing hydrogen peroxide, a peroxidase, a phenol derivative and a plurality of luminescent particles each having a binding functional group.
(2) A second step (S1002) of aggregating the plurality of luminescent particles through the binding of the binding functional groups of the luminescent particles based on a reaction of the hydrogen peroxide, the peroxidase and the phenol derivative in the liquid sample, which occurs when the target substance is present in the liquid sample.
(3) A third step (S1003) of obtaining a value related to fluorescence anisotropy of the liquid sample in the second step.
(4) A fourth step (S1004) of detecting the target substance based on a value related to fluorescence anisotropy.

(Measurement Principle 1: Measurement of Peroxidase Activity)

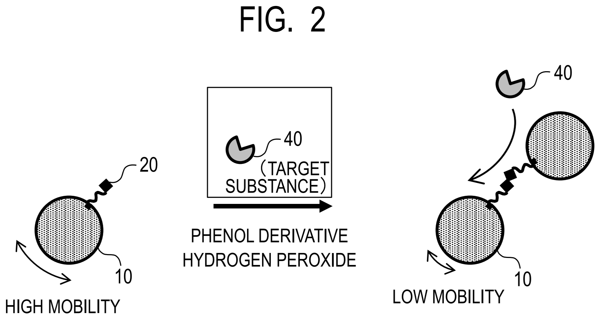

The measurement principle of the present disclosure is described with reference to Fig. 2 by a method of measuring the activity of a peroxidase as an example of an oxidoreductase.

A peroxidase 40, which is the target substance, catalyzes crosslinking through the binding of binding functional groups 20 (functional groups each having a binding ability) of luminescent particles 10 by an oxidation reaction in the presence of specified amounts of hydrogen peroxide and a phenol derivative (including phenol). In other words, the binding functional group 20 of a first luminescent particle out of the luminescent particles 10 binds to the binding functional group of a second luminescent particle different from the first luminescent particle out of the luminescent particles 10 based on a reaction of the hydrogen peroxide, the peroxidase 40 and the phenol derivative. Thus, the plurality of luminescent particles 10 aggregates via the binding functional groups 20. The binding functional group of the first luminescent particle and the binding functional group of the second luminescent particle, which bind to each other here, may be the same kind of functional group or different kinds of functional groups as long as the groups bind to each other based on the reaction of the hydrogen peroxide, the peroxidase 40 and the phenol derivative. In the case of the same kind of functional group, for example, a thiol group is preferrable as the binding functional group. The luminescent particles 10 before the addition of the peroxidase 40 are dispersed in the solution and have high mobility. However, when the peroxidase 40 is added, a crosslinked (also referred to as "aggregated") structure is formed between the luminescent particles 10 in accordance with its activity, and the mobility of the luminescent particles 10 decreases. The decrease in mobility is observed as an increase in value related to fluorescence anisotropy of the luminescent particles 10 (hereinafter described by using the degree of fluorescence polarization as an example of the value related to fluorescence anisotropy). Accordingly, the activity of the peroxidase 40 in the specimen may be determined by measuring the degree of fluorescence polarization of the luminescent particles 10.

In actuality, a large number of luminescent particles 10 are present in the solution, and the proportion of the luminescent particles 10 that aggregate increases in accordance with the activity of the peroxidase 40 in the specimen. Here, the degree of fluorescence polarization obtained by the fluorescence polarization measurement is the average value of the entire solution, that is, the entire luminescent particles 10, and hence the proportion of the luminescent particles 10 that aggregate and the degree of fluorescence polarization correlate with each other.

For example, the peroxidase activity of the specimen may be determined from the measurement result of the degree of fluorescence polarization of the solution based on a calibration curve for a degree of fluorescence polarization and activity obtained in advance by using a peroxidase having known activity.

(Measurement Principle 2: Measurement of Glucose Oxidase Activity)

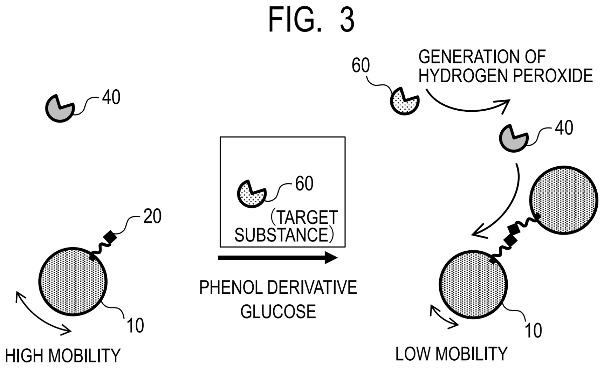

In addition to the peroxidase, the enzyme activity of an oxidase may be measured by a similar method. For example, description is made by taking a glucose oxidase as an example (Fig. 3). The measurement of oxidase activity of the present disclosure utilizes a cascade reaction of two kinds of enzymes, a glucose oxidase 60 and the peroxidase 40. The glucose oxidase 60, which is the target substance, can aggregate the luminescent particles 10 in the presence of specified amounts of glucose, the peroxidase 40 and a phenol derivative.

This principle is the same as Measurement Principle 1 for peroxidase activity described above except that glucose is converted into hydrogen peroxide depending on the activity of the glucose oxidase 60.

That is, glucose is converted into hydrogen peroxide depending on the activity of the glucose oxidase 60. The hydrogen peroxide activates the peroxidase 40 to aggregate the luminescent particles 10. Accordingly, the mobility of the luminescent particles 10 changes depending on the activity of the glucose oxidase 60. The glucose oxidase activity of the specimen may be determined from the measurement result of the degree of fluorescence polarization of the solution based on a calibration curve for a degree of fluorescence polarization and activity obtained in advance by using a glucose oxidase having known activity.

(Measurement Principle 3: Measurement of Hydrogen Peroxide Concentration)

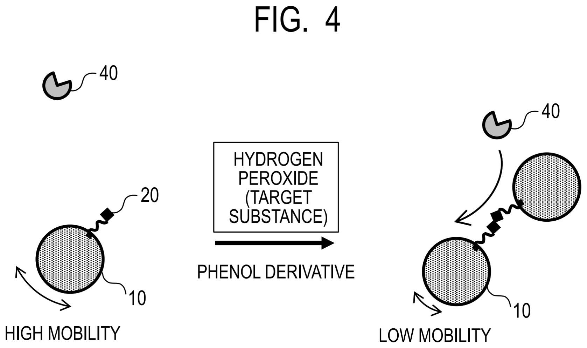

In the present disclosure, the concentration of hydrogen peroxide, which is an example of a peroxidase enzyme reaction-related substance, may also be measured by a similar method (Fig. 4). For example, the hydrogen peroxide, which is the target substance, can aggregate the luminescent particles 10 in the presence of specified amounts of the peroxidase 40 and a phenol derivative.

By measuring the change in mobility of the luminescent particles 10 associated with the change in activity of the peroxidase 40 depending on the amount of the hydrogen peroxide, which is the target substance, by the fluorescence polarization measurement, it becomes possible to measure the hydrogen peroxide concentration. The hydrogen peroxide concentration may be determined from the measurement result of the degree of fluorescence polarization of the solution based on a calibration curve for a degree of fluorescence polarization and a hydrogen peroxide concentration obtained in advance by using a known concentration of hydrogen peroxide.

(Measurement Principle 4: Measurement of Glucose Concentration)

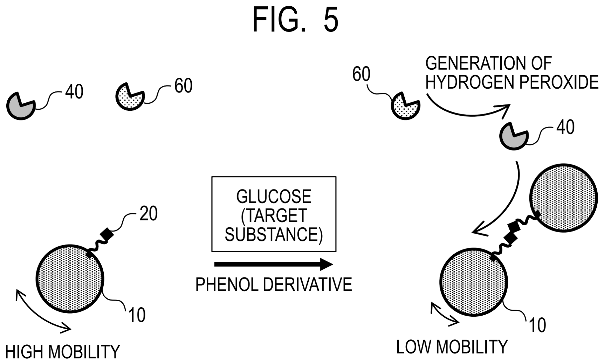

In the present disclosure, the concentration of glucose, which is an example of a peroxidase enzyme reaction-related substance, may also be measured by a similar method (Fig. 5). For example, the glucose, which is the target substance, can aggregate the luminescent particles 10 in the presence of specified amounts of the glucose oxidase 60, the peroxidase 40 and a phenol derivative.

Similarly to the measurement principle of glucose oxidase activity described in Measurement Principle 2, the glucose concentration may be measured by utilizing the cascade reaction of the glucose oxidase 60 and the peroxidase 40. The luminescent particles 10 are caused to coexist in the solution as probe molecules for fluorescence polarization measurement. By measuring the change in mobility of the luminescent particles 10 associated with the change in activity of the peroxidase 40 depending on the amount of the glucose, which is the target substance, by the fluorescence polarization measurement, it becomes possible to measure the glucose concentration. The glucose concentration may be determined from the measurement result of the degree of fluorescence polarization of the solution based on a calibration curve for a degree of fluorescence polarization and a glucose concentration obtained in advance by using a known concentration of glucose.

(Measurement Principle 5: Measurement of Phenol Derivative Concentration)

In the present disclosure, the concentration of a phenol derivative, which is an example of a peroxidase enzyme reaction-related substance, may also be measured by a similar method. For example, the phenol derivative, which is the target substance, can aggregate the luminescent particles 10 in the presence of specified amounts of the peroxidase 40 and hydrogen peroxide.

By detecting the change in mobility of the luminescent particles 10 associated with the change in degree of aggregation of the luminescent particles 10 by the peroxidase 40 depending on the amount of the phenol derivative, which is the target substance, through use of the measurement of a degree of fluorescence polarization, it becomes possible to measure the phenol derivative concentration. The phenol derivative concentration may be determined from the measurement result of the degree of fluorescence polarization of the solution based on a calibration curve for a degree of fluorescence polarization and a phenol derivative concentration obtained in advance by using a known concentration of a phenol derivative.

(Specific Description of Measurement Method)

An example of the present disclosure includes the following steps.

A method of detecting a target substance that is a substance related to an enzyme reaction includes: a step (first step) of mixing a specimen liquid that may contain the target substance and a reagent to obtain a liquid sample containing hydrogen peroxide, a peroxidase, a phenol derivative and a plurality of luminescent particles each having a binding functional group; a step (second step) of aggregating the plurality of luminescent particles through the binding of the binding functional groups of the luminescent particles based on a reaction of the hydrogen peroxide, the peroxidase and the phenol derivative in the liquid sample, which occurs when the target substance is present in the liquid sample; and a step (third step) of obtaining a value related to fluorescence anisotropy of the liquid sample in the second step.

The above-mentioned method of detecting a target substance may include a step (fourth step) of detecting the target substance based on the value related to fluorescence anisotropy. Each step is described in more detail below.

(First Step)

In the present disclosure, in the first step, a specimen liquid that may contain the target substance and a reagent are mixed to obtain a liquid sample containing hydrogen peroxide, a peroxidase, a phenol derivative and a plurality of luminescent particles each having a binding functional group.

A method of mixing the specimen liquid that may contain the target substance and the reagent may include adding the reagent to the specimen liquid, may include adding the specimen liquid to the reagent, or may include extracting and mixing each of the specimen liquid and the reagent.

(Second Step)

In the present disclosure, the second step is a step of forming a crosslinked (also referred to as "aggregated") structure between the plurality of luminescent particles using an enzyme in the liquid sample. For example, by a reaction of an oxidoreductase which is the target substance, a phenol derivative and hydrogen peroxide, the binding functional groups of the plurality of luminescent particles bind to each other, and a crosslinked structure of the plurality of luminescent particles is formed.

In the case where the target substance is hydrogen peroxide, which is an example of an enzyme reaction-related substance, the hydrogen peroxide, which is the target substance, is subjected to a reaction with a solution containing a peroxidase, a phenol derivative and luminescent particles each having a binding functional group, to cause an aggregation reaction of the luminescent particles through the binding of the binding functional groups of the particles. Here, the order of the reactions is not particularly limited, but it is preferrable that the hydrogen peroxide, which is the target substance, be added last because the aggregation between the particles proceeds with the hydrogen peroxide as a trigger.

In the case where the target substance is glucose, which is an example of an enzyme reaction-related substance, the glucose, which is the target substance, is subjected to a reaction with a solution containing a glucose oxidase, a peroxidase, a phenol derivative and luminescent particles each having a binding functional group, and an aggregation reaction of the luminescent particles is performed. Here, the order of the reactions is not particularly limited, but it is preferrable that the glucose, which is the target substance, be added last because the aggregation between the luminescent particles is formed with the glucose as a trigger.

In the case where the target substance is a phenol derivative, which is an example of an enzyme reaction-related substance, the phenol derivative, which is the target substance, and hydrogen peroxide are subjected to a reaction with a solution containing a peroxidase and luminescent particles, and an aggregation reaction of the luminescent particles is performed. Here, the order of the reactions is not particularly limited, but it is preferrable that the hydrogen peroxide be added last because the aggregation between the particles is generated with the hydrogen peroxide as a trigger.

(Liquid Sample)

The liquid sample that may be used in the present disclosure may be any liquid (also referred to as "specimen liquid") that contains a component for measuring the activity or concentration of the target substance and also contains the target substance.

In the present disclosure, the target substance is a peroxidase enzyme reaction-related substance.

The specimen liquid is, for example, a body fluid, such as blood, urine or saliva, containing the target substance, a buffer solution containing the target substance, a culture liquid or tissue extract of cells or microorganisms containing the target substance, drinking water containing the target substance, river water containing the target substance or a waste liquid containing the target substance.

The specimen liquid may contain hydrogen peroxide. The hydrogen peroxide promotes the reaction of a peroxidase, but various kinds of measurement may be performed by appropriately adjusting the concentrations and blending ratios of the peroxidase, a phenol derivative, luminescent particles and the like, and conditions for measuring the degree of fluorescence polarization.

(Oxidoreductase)

In the present disclosure, an oxidoreductase is preferrable as an enzyme to be used in the reaction. The oxidoreductase is an enzyme that catalyzes a redox reaction. Examples of the oxidoreductase include an oxidase, a dehydrogenase, a reductase and an oxygenase. Specific examples thereof include a peroxidase, various oxidases, catalase, glucose dehydrogenase, lactate dehydrogenase, pyruvate dehydrogenase, alcohol dehydrogenase and thioredoxin reductase. Further, examples of the peroxidase include horseradish peroxidase (HRP), myeloperoxidase, cytochrome P450 and manganese peroxidase. Examples of the oxidase include glucose oxidase, lactate oxidase, pyruvate oxidase, cholesterol oxidase, alcohol oxidase, amino acid oxidase and choline oxidase.

(Enzyme Reaction-related Substance)

The enzyme reaction-related substance in the present disclosure is a substance related to a peroxidase enzyme reaction, and may be any substance whose amount or activity may be measured by the measurement method of the present disclosure. That is, the enzyme reaction-related substance may be any substance that affects the activity of a peroxidase. For example, the enzyme reaction-related substance is a substrate for an oxidoreductase, hydrogen peroxide or a phenol derivative. The amount or activity of such enzyme reaction-related substance changes the degree of fluorescence polarization obtained by the fluorescence polarization measurement of the present disclosure. Examples of the substrate for an oxidoreductase include glucose, lactic acid, pyruvic acid and cholesterol.

(Luminescent Particles)

The luminescent particles each having a binding functional group to be used in the present disclosure may be any luminescent particles which are nanoparticles each containing a luminescent substance and whose degree of fluorescence polarization for luminescence may be measured by fluorescence polarization measurement. In addition, the luminescent particles according to the present disclosure may be luminescent particles between which crosslinking can be formed by the action of an enzyme reaction. The mode of crosslinking between the luminescent particles may be a chemical bond, such as a covalent bond, an ionic bond or a coordinate bond. For chemical bonding, the luminescent particles each have a binding functional group, such as a thiol group, an amino group, a carboxy group or a maleimide group. The binding functional group is preferably a thiol group because the reaction proceeds mildly and rapidly, in particular.

The luminescent particles of the present disclosure are nanoparticles, and the average particle diameter that is the average of the diameters of the particles is a diameter of 1 nm or more to 1,000 nm or less, preferably 25 nm or more to 500 nm or less, and the average particle diameter is more preferably 50 nm or more to 300 nm or less. When the average particle diameter is more than 500 nm, the degree of fluorescence polarization before crosslinking between the particles may increase, and the amount of change in degree of fluorescence polarization after aggregation between the particles may decrease. In addition, when the average particle diameter is less than 25 nm, the content of the luminescent substance in the nanoparticles decreases, and the luminescence intensity per particle decreases. As a result, the measurement sensitivity and accuracy may decrease, and hence luminescent particles of 50 nm or more are preferrable. The size of the luminescent particles may be examined by dynamic light scattering (DLS) measurement.

As a particle material (also referred to as "matrix material" or "main component") for the luminescent particles, for example, nanoparticles of a synthetic polymer, such as polystyrene or polymethacrylate, inorganic nanoparticles, such as silica, titanium oxide or iron oxide, or a natural polymer, such as dextran or casein, may be used. Of those, a synthetic polymer is preferrable as the particle material for the luminescent particles according to the present disclosure because the synthetic polymer has a specific gravity that allows dispersion in water, is stable, and has high productivity, and polystyrene particles excellent in particle size controllability are particularly preferrable.

In addition, it is preferrable that the surfaces of the particles be hydrophilized. This is because by hydrophilizing the surfaces of the particles, non-specific aggregation between the particles can be suppressed, and non-specific adsorption of the particles to a measurement container (e.g., plastic cuvette or quartz glass) can also be suppressed. In the measurement method using the fluorescence polarization measurement of the present disclosure, a change in mobility of the luminescent particles irrespective of the activity or amount of an enzyme or the concentration of an enzyme reaction-related substance causes a decrease in accuracy or precision of the measurement of the target substance. Accordingly, it is required to avoid non-specific aggregation between the particles and non-specific adsorption of the particles to the container. For this reason, it is preferable that the surfaces of the particles be coated with a hydrophilic polymer. For example, a polymer, such as polyvinylpyrrolidone, polyethylene glycol, polyhydroxymethacrylate, dextran or bovine serum albumin, is suitably used. In addition, as described above, the luminescent particles are crosslinked between the plurality of particles. In order to promote crosslinking between the luminescent particles, a binding functional group may be appropriately introduced into each of the surfaces of the luminescent particles. For example, when a binding functional group, such as a thiol group, an amino group, a carboxy group or a maleimide group, is present on each of the surfaces of the luminescent particles, the binding between the luminescent particles each having a thiol group, a carboxy group, an amino group or the like can be promoted. This is because an electrostatic bond, a disulfide bond, a bond between a thiol group and a maleimide group or the like occur between the luminescent particles. Those bonds include, for example, a bond between thiol groups (disulfide bond) promoted by an oxidation reaction, and in the case of using a phenol derivative having a thiol group or a maleimide group, an oligomer having a large number of thiol groups or maleimide groups is generated, and the groups act as a binder to bind the luminescent particles each having a thiol group or a maleimide group. Accordingly, as a preferrable example of the surfaces of the luminescent particles, one that is coated with a hydrophilic polymer such as polyvinylpyrrolidone and further has a thiol group introduced is preferrable.

The luminescent particles to be used in the present disclosure do not specifically bind to the target substance. That is, the luminescent particles do not serve as a substrate for an enzyme, which is an example of the target substance of the present disclosure. Similarly, an enzyme reaction-related substance (e.g., glucose or hydrogen peroxide), which is an example of the target substance of the present disclosure, also does not specifically bind to the luminescent particles. By adopting such mode, the luminescent particles can function independently as a probe in the fluorescence polarization measurement. An immunoassay using antibody-bound luminescent particles in which an antibody against an enzyme is bound to the luminescent particles is known for detecting an enzyme as a target substance, but the measurement of a trace amount of an enzyme depends on the binding affinity of an antibody, and the construction of a high-sensitivity assay involves complicated work. In addition, in an immunoassay, it is extremely difficult to evaluate the activity of an enzyme. Meanwhile, in the method of the present disclosure, by adding sufficient amounts of a substrate and luminescent particles, it becomes possible to increase the sensitivity of the measurement of the amount or activity of an enzyme depending on a reaction time.

The luminescent particles to be used in the present disclosure are characterized by containing a luminescent substance. Herein, the luminescent substance may be any substance that can be incorporated into a particle and can be measured by fluorescence polarization measurement. The luminescent substance may be incorporated into the particle surface or the inside of the particle, and is preferably incorporated into the inside of the particle. This is because interaction between the luminescent substance and contaminants or water molecules in a solution may occur on the particle surface to affect the dispersibility of the luminescent particles and fluorescence polarization measurement. Examples of the luminescent substance include a fluorescent dye, such as a fluorescein derivative, a rhodamine derivative or a cyanine derivative, and a rare earth luminescent complex, such as a europium complex or a terbium complex. The europium complex is suitable as the luminescent substance to be incorporated into the luminescent particles according to the present disclosure because its emission wavelength and intensity are not easily affected by the surroundings and its emission lifetime is long.

Specific examples of the europium complex include [tris(2-thenoyltrifluoroacetone)bis(triphenylphosphineoxide)europium(III)], [tris(2-thenoyltrifluoroacetone)(triphenylphosphineoxide)(dibenzylsulfoxide)europium(III)], and [tris(2-thenoyltrifluoroacetone)(phenanthroline)europium(III)].

It is preferrable that the content of the luminescent substance in the luminescent particles be higher. This is because the luminescence intensity per particle increases, and as a result, the increase can contribute to higher sensitivity or improved measurement accuracy for measuring the activity or amount of an enzyme or the concentration of an enzyme reaction-related substance of the present disclosure. It is preferrable that the content of the rare earth luminescent complex in the luminescent particles be, for example, 0.001 g or more of the rare earth luminescent complex per 1 g of the particles. With this content, the luminescence intensity per particle is sufficiently high and stable luminescence can be obtained. In addition, the content of the luminescent substance may be measured by elemental analysis using the specific element of the luminescent substance as an indicator, and for example, the content of the rare earth luminescent complex may be calculated from the quantification of the rare earth luminescent complex by inductively coupled plasma (ICP) emission measurement.

(Phenol Derivative)

The phenol derivative to be used in the present disclosure is not particularly limited as long as the phenol derivative is a phenol compound having a phenolic hydroxy group and is capable of generating a phenoxy radical by the action of a peroxidase. An example thereof is a low-molecular-weight phenol compound having at least one phenolic hydroxy group and having a molecular weight of 1,000 or less. The phenol derivative is preferably a low-molecular-weight phenol compound having 1 to 6 phenolic hydroxy groups and having a molecular weight of 500 or less. Examples thereof include phenol, catechol, resorcinol, hydroquinone, tyramine and hydrochlorides thereof, serotonin, N-glycyl-L-tyrosine (Gly-Tyr), 3-(4-hydroxyphenyl)propionic acid, methyl 3-(4-hydroxyphenyl)propionate, 4-hydroxyphenylacetic acid, 3-hydroxyphenylacetic acid, p-coumaric acid, caffeic acid, dopamine, 6-hydroxydopamine and norepinephrine. From the viewpoints of water solubility and reaction stability, tyramine, tyramine hydrochloride, phenol, glycyl-L-tyrosine, resorcinol and serotonin are preferably used. In the present disclosure, the phenol derivative may be referred to as phenols.

(Hydrogen Peroxide)

In the present disclosure, hydrogen peroxide is used for the aggregation reaction of the luminescent particles by HRP. The mechanism of aggregation between particles by a peroxidase utilized in the method of the present disclosure is presumed to be through a phenol derivative generated by the peroxidase, as disclosed in U.S. Patent Application Publication No. 2017/0239356 (International Publication No. WO2015/159995). For example, when a thiol group is used as the binding functional group of each of the luminescent particles, a radical transfer reaction from a phenoxy radical generated in a reaction solution to the thiol group occurs by the action of the phenol derivative on HRP, and a thiol radical rapidly undergoes a disulfide bond reaction with another thiol group (that is, aggregation between the luminescent particles). In addition, it has been reported that hydrogen peroxide is generated during the disulfide bond reaction, and the catalytic cycle of HRP proceeds spontaneously without the addition of hydrogen peroxide from the outside. As a result, it has been reported that the oxidation of SH groups (disulfide bond reaction) by HRP is promoted under the conditions of a short period of time and a low HRP concentration. However, as is described later in Example 1 and Comparative Example 2, it has been found that the addition of hydrogen peroxide from the outside is essential for the measurement by the fluorescence polarization measurement of the present disclosure. This is because the present disclosure aims at rapid measurement, and specifically, it is required to greatly change the degree of fluorescence polarization of the luminescent particles as a probe in a reaction time of about several minutes. Under the condition that hydrogen peroxide is not used, which is shown in the related art (Comparative Example 2), a sufficient increase in degree of fluorescence polarization was not observed. Accordingly, it is conceived that the addition of hydrogen peroxide is essential for the measurement method of the present disclosure to be established.

When the activity of glucose oxidase is measured, the addition of hydrogen peroxide from the outside is not essential because hydrogen peroxide is generated by the action of glucose oxidase, but an appropriate amount of hydrogen peroxide may be added to accelerate the crosslinking reaction between the luminescent particles and obtain a large signal.

(Third Step)

In the present disclosure, the third step is a step of obtaining a value related to fluorescence anisotropy of the liquid sample in the second step. The fluorescence polarization measurement may be performed by a known method, and various commercially available measurement apparatus may be used for that purpose. A method of obtaining the value related to fluorescence anisotropy is also called fluorescence polarization measurement or fluorescence depolarization measurement, but in the present specification, the fluorescence polarization measurement and the fluorescence depolarization measurement are synonymous. Herein, the value related to fluorescence anisotropy is specifically the degree of fluorescence polarization, but may be a value indicating fluorescence anisotropy determined by calculation from the degree of fluorescence polarization, or may be a value that becomes a value indicating the degree of fluorescence polarization by calculation. The degree of fluorescence polarization (p) and the fluorescence anisotropy (r) have the relationship of the following formula (1).

$$P = 3r/(2+r) \cdots (1)$$

In the present disclosure, an enzyme or an enzyme reaction-related substance that is a target substance may be subjected to fluorescence polarization measurement using the mobility of luminescent particles in a sample solution as an indicator. That is, as a result of the generation of crosslinks between the luminescent particles in the sample solution in accordance with the activity or amount of the enzyme or the amount of the enzyme reaction-related substance, the mobility of the luminescent particles decreases. A feature of the present disclosure is to measure the mobility of the luminescent particles by using the degree of fluorescence polarization. In the fluorescence polarization measurement, the degree of fluorescence polarization (mp) may be used as an indicator.

A feature of the present disclosure is to use, as a probe, luminescent particles each containing a europium complex, which has a long emission lifetime and exhibits polarized luminescence, as a luminescent substance in the particles. A slight change in rotational movement of the luminescent particles in a liquid sample may be captured as a change in polarized luminescence characteristic. Specifically, when the mobility of the luminescent particles is greatly reduced by crosslinking between the luminescent particles, the decrease in rotational Brownian motion of the particles may be captured with high sensitivity as a change in degree of fluorescence polarization.

The timing of the measurement of the degree of fluorescence polarization may be set appropriately. For example, the degree of fluorescence polarization may be measured after the second step. This mode is preferable because the degree of fluorescence polarization only needs to be measured at the time point of the completion of the reaction and the operation is simple. For example, the degree of fluorescence polarization may be measured 5 minutes after the target substance is added to the solution.

Alternatively, the degree of fluorescence polarization of the luminescent particles may be measured before the reaction with the target substance (enzyme or enzyme reaction-related substance) in the second step, and the degree of fluorescence polarization may be set as an initial degree of fluorescence polarization (mp(0)), and then the reaction may proceed and a degree of fluorescence polarization (mp(t)) at a certain time point may be measured again after the crosslinking between the luminescent particles (after the second step), or the degree of fluorescence polarization may be continuously measured at regular time intervals (during the second step to after the second step). That is, by measuring the change in degree of fluorescence polarization over time, the amount of change in degree of fluorescence polarization ($\Delta$mp) may be determined from the difference between the degree of fluorescence polarization (mp(t)) at a certain time point and the initial degree of fluorescence polarization (mp(0)). The change in degree of fluorescence polarization occurs rapidly, and for example, a sufficient $\Delta$mp can be obtained in from about 1 minute to about 10 minutes. A rate of change in degree of fluorescence polarization (dmp/dt) may also be determined.

Measurement conditions for the degree of fluorescence polarization are preferably, for example, as follows: in a liquid at a temperature of from 1°C to 50°C, the viscosity of the liquid is from 0.5 mPa·s to 50 mPa·s. When the luminescent particles are luminescent particles each containing a europium complex, the concentration of the luminescent particles is not limited as long as the luminescence of the luminescent particles can be detected, and the concentration of the luminescent particles may be appropriately selected in accordance with, for example, the amount of the target substance, enzyme, hydrogen peroxide or phenol derivative. It is preferable to measure the degree of fluorescence polarization in the range of from 0.0001 mg/mL to 1.0 mg/mL, but in order to ensure the luminescence intensity from the luminescent particles and avoid the influence of scattering of the luminescent particles, it is preferable to measure the degree of fluorescence polarization in the range of from 0.001 mg/mL to 0.1 mg/mL.

(Fourth Step)

In the present disclosure, the fourth step is a step of detecting a target substance related to an enzyme reaction in the liquid sample based on a value related to fluorescence anisotropy of the luminescent particles in the liquid sample obtained in the third step. More specifically, the fourth step is a step of associating a change in degree of fluorescence polarization with the activity or amount of an enzyme or the amount of an enzyme reaction-related substance.

In the third step, the degree of decrease in mobility of the luminescent particles in accordance with the crosslinking between the luminescent particles is detected as the degree of fluorescence polarization. The degree of decrease in mobility of the luminescent particles depends on the activity or amount of an oxidoreductase or the amount of an enzyme reaction-related substance present in the liquid sample. Accordingly, by associating the degree of fluorescence polarization obtained in the third step with the activity or amount of the oxidoreductase or the amount of the enzyme reaction-related substance in the liquid sample, the activity or amount of the oxidoreductase or the amount of the enzyme reaction-related substance in the liquid sample can be measured. For example, a case in which the degree of fluorescence polarization obtained in the third step is high means that the luminescent particles are aggregated and the ratio of the aggregates is large, and thus the high degree of fluorescence polarization can be associated with high oxidoreductase activity.

The term "measurement method" in the present disclosure includes not only quantitative measurement but also qualitative measurement (detection of the presence or absence of an enzyme or an enzyme reaction-related substance). Accordingly, the association of a value related to fluorescence anisotropy of the luminescent particles with the activity or amount of the oxidoreductase or the amount of the enzyme reaction-related substance in the fourth step may be quantitative or qualitative.

Quantitative association may be performed by, for example, determining a relational expression between enzyme activity and a degree of fluorescence polarization in advance using an enzyme solution having known enzyme activity, and then using this expression to determine the enzyme activity of the target substance from the degree of fluorescence polarization obtained by measuring the target substance. By performing such association, the enzyme activity in the liquid sample can be measured. The enzyme activity measured here may also be determined as an enzyme concentration, a mass, the number of molecules or the like.

In the present disclosure, similarly, a relational expression between the concentration of hydrogen peroxide or glucose and a degree of fluorescence polarization may be determined in advance by using, for example, a hydrogen peroxide solution having a known concentration or a glucose solution having a known concentration. The concentration of the target substance can be determined from the degree of fluorescence polarization obtained by measuring the target substance.

In addition, in the fourth step, the change in degree of fluorescence polarization of the luminescent particles may be handled relatively. That is, the activity or amount of an enzyme or the amount of an enzyme reaction-related substance may be associated relatively, and for example, the value of the degree of fluorescence polarization (mp) obtained by fluorescence polarization measurement may be used. For example, in applications for screening enzymes having high activity, screening may be performed simply based on the value of the degree of fluorescence polarization (mp) or the value of its amount of change ($\Delta$mp) for an enzyme population of the same concentration.

(Reagent for Inspection for detecting Target Substance using Measurement of Degree of Fluorescence Polarization)

According to the present disclosure, there may be provided a reagent for inspection as listed in any one of the following items (1) to (3), utilizing the above-mentioned measurement system.

(1) A reagent for inspection to be used in a method of measuring an enzyme reaction-related substance of a peroxidase in the present disclosure, the reagent including luminescent particles each having a binding functional group, a phenol derivative, and hydrogen peroxide.

The luminescent particles each having a binding functional group and the phenol derivative may be provided in a solution state, a dry state, a frozen state, a lyophilized state and the like. Hydrogen peroxide may be provided in a solution state. As a stabilizer for hydrogen peroxide, conventionally used salicylic acid or the like may be included.

The reagent for inspection of a target substance of the present disclosure may be a single reagent, or may be divided into a plurality of reagents, for example, a first liquid, a second liquid and a third liquid. From the viewpoint of the stability of each reagent, for example, the first liquid includes luminescent particles each having a crosslinking functional group, the second liquid includes a phenol derivative, and the third liquid includes hydrogen peroxide.

In addition, the reagent for inspection of a target substance of the present disclosure may be divided into a solution state and a dry state, and the reagent in the dry state may be subjected to various kinds of measurement by being brought into a solution state with an attached dissolving liquid before the measurement.

(2) A reagent for inspection to be used in a method of measuring hydrogen peroxide that is a target substance according to the present disclosure, the reagent including an oxidoreductase, luminescent particles each having a binding functional group and a phenol derivative.

The oxidoreductase, the luminescent particles each having a binding functional group and the phenol derivative may be provided in a solution state, a dry state, a frozen state, a lyophilized state and the like. In addition, the reagent for inspection of a target substance of the present disclosure may be divided into a plurality of reagents, for example, a first liquid, a second liquid and a third liquid. For example, the first liquid includes an oxidoreductase, the second liquid includes luminescent particles each having a binding functional group, and the third liquid includes a phenol derivative.

(3) A reagent for inspection to be used in a method of measuring glucose that is a target substance according to the present disclosure, the reagent including glucose oxidase, a peroxidase, luminescent particles each having a binding functional group and a phenol derivative.

Glucose oxidase, a peroxidase, luminescent particles each having a binding functional group and a phenol derivative may be provided in a solution state, a dry state, a frozen state, a lyophilized state and the like. In addition, the reagent for inspection of a target substance of the present disclosure may be divided into a plurality of reagents, for example, a first liquid, a second liquid, a third liquid and a fourth liquid. For example, the first liquid includes glucose oxidase, the second liquid includes a peroxidase, the third liquid includes luminescent particles each having a binding functional group, and the fourth liquid includes a phenol derivative.

As a reagent for inspection of a target substance, when the target substance is a peroxidase, the reagent may be a reagent including hydrogen peroxide, a phenol derivative and luminescent particles each having a binding functional group. When the target substance is an oxidase, the reagent may be a reagent including a peroxidase, hydrogen peroxide, a phenol derivative and luminescent particles each having a binding functional group. When the target substance is hydrogen peroxide, the reagent may be a reagent including a peroxidase, a phenol derivative and luminescent particles each having a binding functional group. When the target substance is a substrate for an oxidase, the reagent may be a reagent including an oxidase, a peroxidase, a phenol derivative and luminescent particles each having a binding functional group. When the target substance is a phenol derivative, the reagent may be a reagent including a peroxidase, hydrogen peroxide and luminescent particles each having a binding functional group. When the target substance is a glucose, the reagent may be a reagent including a glucose oxidase, a peroxidase, luminescent particles each having a binding functional group and a phenol derivative.

[Examples]

The present disclosure is described in more detail below by way of Examples. (1: Production of Thiol Group-introduced Luminescent Particle T1)

A luminescent particle to be used in a method of measuring activity or amount of an enzyme or the amount of an enzyme reaction-related substance according to the present disclosure is characterized by having a binding functional group capable of crosslinking between the luminescent particles on its surface. Here, a synthesis example of a luminescent particle having a thiol group as a binding functional group on its surface is shown.

First, polyvinylpyrrolidone (PVP-K30: manufactured by Tokyo Chemical Industry Co., Ltd.) was dissolved in a 2-morpholinoethanesulfonic acid (MES) buffer solution (manufactured by Kishida Chemical Co., Ltd.) having a pH of 7 to prepare a solvent A. Subsequently, [tris(2-thenoyltrifluoroacetone)bis(triphenylphosphineoxide)europium(III)] (manufactured by Central Techno Corporation, hereinafter abbreviated as "Eu(TTA)$_3$(TPPO)$_2$") serving as a europium complex, a styrene monomer (manufactured by Kishida Chemical Co., Ltd.) and 3-methacryloxypropyltrimethoxysilane (manufactured by Tokyo Chemical Industry Co., Ltd., hereinafter abbreviated as "MPS") were mixed to prepare a reaction liquid B. The reaction liquid B was added into a four-necked flask containing the solvent A, and the mixture was stirred with a mechanical stirrer set to 300 rpm.

After 15 minutes of stirring under a nitrogen flow condition, the temperature of an oil bath that had been prepared was set to 70°C, and the nitrogen flow was performed for an additional 15 minutes. After the mixed liquid had been heated and stirred, an aqueous solution having dissolved therein potassium persulfate (hereinafter abbreviated as "KPS") (manufactured by Sigma-Aldrich) was added into the reaction solution, and emulsion polymerization was performed for 20 hours. After the polymerization reaction, the resultant suspension was subjected to ultrafiltration with about 4 L of ion-exchanged water through use of an ultrafiltration membrane having a molecular weight cutoff of 100K to wash the product, to thereby provide a dispersion of luminescent particles.

An aliquot of the dispersion of the luminescent particles obtained by the emulsion polymerization was taken and added to an aqueous solution having dissolved therein 1% by mass of Tween 20 (manufactured by Kishida Chemical Co., Ltd.). After 10 minutes of stirring, a silane coupling agent, X12-1135 (manufactured by Shin-Etsu Chemical Co., Ltd.), was added, and the mixture was stirred overnight (a mass ratio among the particles, pure water and X12-1135 loaded was 1:300:2). After the stirring, the dispersion was centrifuged, the supernatant was removed, and the precipitate was redispersed with pure water. The operations of centrifugation and redispersion were performed three or more times to wash the product. The precipitate after the washing was redispersed in pure water. Thus, luminescent particles 1 were obtained.

Next, a 0.25 mL aliquot of the particle dispersion of the luminescent particles 1 (particle concentration: 1.2% by mass) was taken, and the solvent was replaced with 1.6 mL of a MES buffer solution having a pH of 6.0. To the MES buffer solution in which the particles were dispersed, 1-[3-(dimethylamino)propyl]-3-ethylcarbodiimide and N-hydroxysulfosuccinimide sodium salt were added at 0.5% by mass, and the mixture was subjected to a reaction at 25°C for 1 hour. After the reaction, the dispersion was washed with a MES buffer solution having a pH of 5.0, 2-aminoethanethiol hydrochloride was added thereto, and the mixture was subjected to a reaction at 25°C for 2 hours to introduce 2-aminoethanethiol into the surface of each of the particles. After the introduction, the particles were washed with a Tris buffer solution having a pH of 8. After that, the particles were washed with a phosphate buffer solution to provide thiol group-modified luminescent particles having a concentration of 0.3% by mass (hereinafter referred to as "luminescent particles T1"). The aqueous solution containing the luminescent particles T1 is referred to as "luminescent particle T1 solution."

The introduction of the thiol group into each of the particles was recognized by adding Ellman's reagent to the luminescent particle T1 solution, centrifuging the luminescent particle T1 solution, and then measuring the absorbance (410 nm) of the supernatant. The presence of the thiol group on the surface of each of the particles was able to be recognized by an increase in the absorbance compared to that before the addition of Ellman's reagent.

(2: Production of Phenol Derivative Solution)

A tyramine hydrochloride phosphate buffer solution having a final concentration of 40 mM was prepared by dissolving tyramine hydrochloride in a phosphate buffer solution (containing 0.01% Tween 20).

(3: Production of Hydrogen Peroxide Aqueous Solution)

A 3.5% hydrogen peroxide aqueous solution was prepared by diluting a hydrogen peroxide aqueous solution (35%) with water.

(4: Production of Specimen Solution containing Peroxidase as Target Substance)

A specimen solution (also referred to as "target substance") having a final concentration of 2 mg/mL (0.05 mM) was prepared by dissolving horseradish peroxidase (HRP) in water.

With the above-mentioned sample production examples, examples of the fluorescence polarization measurement of luminescent particles by a peroxidase enzyme reaction are shown in Examples 1 to 6 to be described later.

In a reaction solution containing a europium complex, the surfaces of particles are hydrophilized with Tween 20 and a silane coupling agent (luminescent particles 10), and a thiol group (the binding functional group 20 of the luminescent particle) is further added with 2-aminoethanethiol hydrochloride. The luminescent particles form disulfide crosslinked aggregates by HRP (peroxidase 40), tyramine hydrochloride (phenol derivative) and hydrogen peroxide. Thus, the mobility of the luminescent particles changes and is detected as a change in degree of fluorescence polarization. Further, in Example 6 to be described later, a measurement example using glucose oxidase (glucose oxidase 60) as a source of hydrogen peroxide is shown.

(Example 1: Fluorescence Polarization Measurement of Peroxidase)

Water, the luminescent particle T1 solution and tyramine hydrochloride were mixed at the ratios shown in Table 1.

A target substance (HRP) was added to the aqueous solution, followed by thorough stirring.

The resultant solution was transferred to a 96-well microplate. Next, a 3.5% hydrogen peroxide aqueous solution was added to the solution in the wells, and the mixture was stirred well. Then, the microplate was set in a fluorescence polarization measurement apparatus (Nivo multimode microplate reader). A degree of fluorescence polarization (mp) was measured after 5 minutes.

The conditions of the fluorescence polarization measurement apparatus are as follows:

Mode: FP kinetics;
Excitation light: center wavelength of 355 nm/width of 40 nm;
Emission filter: (S) center wavelength of 615 nm/width of 8 nm, (P) center wavelength of 615 nm/width of 8 nm;
Dichroic mirror: D400;
Measurement time: 1,000 ms;
Z-focus: 5 mm;
Measurement spot size: 2 mm on excitation side, 4 mm on emission side;
Flash energy: low (10); and
PMT HV: 1,000.

(Comparative Example 1: Fluorescence Polarization Measurement of Sample free of Peroxidase)

A sample was prepared in the same manner as in Example 1 except that 20 μL of water was added instead of the target substance HRP. A degree of fluorescence polarization (mp) was measured in the same manner as in Example 1.

(Comparative Example 2: Fluorescence Polarization Measurement of Sample free of Hydrogen Peroxide)

A sample was prepared in the same manner as in Example 1 except that the 3.5% hydrogen peroxide aqueous solution was not added. A degree of fluorescence polarization (mp) was measured in the same manner as in Example 1.

(Comparative Example 3: Fluorescence Polarization Measurement of Sample free of Phenol Derivative)

A sample was prepared in the same manner as in Example 1 except that tyramine hydrochloride was not added. A degree of fluorescence polarization (mp) was measured in the same manner as in Example 1.

The results of Example 1 and Comparative Examples 1 to 3 are shown in Table 1.

In the sample free of HRP (Comparative Example 1), the degree of fluorescence polarization was 63.0, but in the sample containing HRP, the degree of fluorescence polarization was as large as 80.0. This means that in Example 1, the mobility of the luminescent particles in the sample solution decreased, and it was found that aggregation of the luminescent particles with the activity of HRP occurred. From this Example, it was found that the presence of the peroxidase aggregated the luminescent particles and increased the degree of fluorescence polarization.

When hydrogen peroxide was not added (Comparative Example 2), the degree of fluorescence polarization was as small as 63.0 in the same manner as in Comparative Example 1. It was recognized that the luminescent particles did not aggregate. From this Comparative Example, it was found that the addition of the hydrogen peroxide was required for measuring the peroxidase.

When the phenol derivative (tyramine hydrochloride) was not added (Comparative Example 3), the degree of fluorescence polarization was as small as 62.8. From this Comparative Example, it was found that the addition of a phenol derivative (tyramine hydrochloride) is required for rapid measurement of peroxidase by the fluorescence polarization measurement.

Table 1

| | Water | Luminescent particles T1 | Phenol derivative (tyramine hydrochloride) | Hydrogen peroxide aqueous solution | Target substance HRP | Degree of fluorescence polarization (mp) |
|---|---|---|---|---|---|---|
| Stock solution concentration | | 0.08 mg/mL | 40 mM | 3.5% solution | 2 mg/mL | |
| | Reagent composition (μL) | | | | Target substance (μL) | |
| Example 1 | 100 | 10 | 30 | 5 | 20 | 80.0 |
| Comparative Example 1 | 100 | 10 | 30 | 5 | 0 | 63.0 |
| Comparative Example 2 | 100 | 10 | 30 | 0 | 20 | 63.0 |
| Comparative Example 3 | 100 | 10 | 0 | 5 | 20 | 62.8 |

(Example 2: Fluorescence Polarization Measurement of Peroxidase)

Water, the luminescent particle T1 solution and tyramine hydrochloride were mixed at the ratios shown in Table 2. Different concentrations of a target substance (HRP) were each added to the aqueous solution, followed by thorough stirring. Solutions different from each other in HRP activity were prepared by preparing the target substance (HRP) from HRP having known activity.

Those solutions were each transferred to a 96-well microplate. Next, a 3.5% hydrogen peroxide aqueous solution was added to the solution in the wells, and the mixture was stirred well. Then, a degree of fluorescence polarization (mp) was measured in the same manner as in Example 1.

The results of Example 2 are shown in Table 2. As the concentration or activity of HRP increased, the degree of fluorescence polarization increased. It is conceived that crosslinking between the luminescent particles was caused in accordance with the activity of HRP, to decrease the mobility of the luminescent particles. It was found that by using the relational expression (calibration curve) between the amount or activity of the peroxidase and the degree of fluorescence polarization obtained from this Example, the activity or concentration of a peroxidase having an unknown concentration was able to be determined by measuring its degree of fluorescence polarization.

Table 2

| | Water | Luminescent particles T1 | Phenol derivative (tyramine hydrochloride) | Hydrogen peroxide aqueous solution | Target substance HRP (20 µL) | | Degree of fluorescence polarization (mp) |
|---|---|---|---|---|---|---|---|
| Stock solution concentration | | 0.08 mg/mL | 40 mM | 3.5% solution | | | |
| | Reagent composition (µL) | | | | Final concentration of target substance (µg/mL) | Activity of target substance (U/mL) | |
| Example 2 | 100 | 10 | 30 | 5 | 0 | 0 | 63.3 |
| | 100 | 10 | 30 | 5 | 12.1 | 2.4 | 67.7 |
| | 100 | 10 | 30 | 5 | 60.6 | 12.1 | 74.0 |
| | 100 | 10 | 30 | 5 | 242.4 | 48.5 | 84.6 |

(Example 3: Fluorescence Polarization Measurement of Peroxidase Activity)

Water, the luminescent particle T1 solution and tyramine hydrochloride were mixed at the ratios shown in Table 3. A target substance (HRP) having the same activity was added to the aqueous solution, followed by thorough stirring. A sample obtained by further adding water thereto and another sample obtained by adding a sodium azide aqueous solution, which was an enzyme activity inhibitor, thereto were prepared. Those solutions were each transferred to a 96-well microplate. Next, a 3.5% hydrogen peroxide aqueous solution was added to the solution in the wells, and the mixture was stirred well. Then, a degree of fluorescence polarization (mp) was measured in the same manner as in Example 1.

The results of Example 3 are shown in Table 3. In the sample in which the sodium azide aqueous solution was added, the degree of fluorescence polarization was small as compared to that of the sample in which the sodium azide aqueous solution was not added. That is, it was found that the decrease in enzyme activity of HRP was able to be measured by the fluorescence polarization measurement.

Table 3

| | Water | Luminescent particles T1 | Phenol derivative (tyramine hydrochloride ) | Hydrogen peroxide aqueous solution | Target substance HRP (10 µL) | Enzyme activity inhibitor sodium azide aqueous solution (0.09%) | Degree of fluorescence polarization (mp) |
|---|---|---|---|---|---|---|---|
| Stock solution concentration | | 0.08 mg/mL | 40 mM | 3.5% solution | | | |
| | Reagent composition (µL) | | | | Final concentration of target substance (µg/mL) | | |
| Example 3 | 100 | 10 | 30 | 1 | 124.2 | Absent | 79.2 |
| | 100 | 10 | 30 | 1 | 124.2 | Present | 69.4 |

(Example 4: Fluorescence Polarization Measurement of Hydrogen Peroxide Concentration)

Water, the luminescent particle T1 solution, tyramine hydrochloride and HRP were mixed at the ratios shown in Table 4. The resultant solution was transferred to a 96-well microplate. Next, various concentrations of hydrogen peroxide were each added as a target substance to the solution in the wells, followed by thorough stirring. Then, a degree of fluorescence polarization (mp) was measured in the same manner as in Example 1.

The results of Example 4 are shown in Table 4. As the concentration of hydrogen peroxide increased, the degree of fluorescence polarization increased. It is conceived that the mobility of the luminescent particles decreased in accordance with the amount of hydrogen peroxide.

It was found that by using the relational expression (calibration curve) between the amount (concentration) of hydrogen peroxide and the degree of fluorescence polarization obtained from this Example, the concentration of a hydrogen peroxide aqueous solution having an unknown concentration was able to be determined by measuring its degree of fluorescence polarization.

Table 4

| | Water | Luminescent particles T1 | Phenol derivative (tyramine hydrochloride) | HRP | Target substance hydrogen peroxide (5 μL) | Degree of fluorescence polarization (mp) |
|---|---|---|---|---|---|---|
| Stock solution concentration | | 0.08 mg/mL | 40 mM | 2 mg/mL | | |
| | Reagent composition (μL) | | | | Final concentration of target substance (%) | |
| Example 4 | 100 | 10 | 30 | 20 | 0.00000 | 63.4 |
| | 100 | 10 | 30 | 20 | 0.00212 | 70.0 |
| | 100 | 10 | 30 | 20 | 0.01061 | 74.1 |
| | 100 | 10 | 30 | 20 | 0.02121 | 78.8 |
| | 100 | 10 | 30 | 20 | 0.10606 | 83.3 |

(Example 5: Fluorescence Polarization Measurement of Glucose Oxidase)

Water, the luminescent particle T1 solution, tyramine hydrochloride, HRP and glucose were mixed at the ratios shown in Table 5. The resultant solution was transferred to a 96-well microplate. Next, various concentrations of glucose oxidase (abbreviated as "GOX") were each added as a target substance to the solution in the wells, followed by thorough stirring. Then, a degree of fluorescence polarization (mp) was measured in the same manner as in Example 1 except that the degree of fluorescence polarization was measured after 20 minutes.

The results of Example 5 are shown in Table 5. As the concentration of GOX increased, the degree of fluorescence polarization increased. It is conceived that hydrogen peroxide was produced from glucose in the solution by the target substance GOX, and the hydrogen peroxide triggered the activation of the peroxidase to aggregate the particles, resulting in the increase in degree of fluorescence polarization.

It was found that by using the relational expression (calibration curve) between the GOX concentration and the degree of fluorescence polarization obtained from this Example, the concentration of GOX having an unknown concentration was able to be determined by measuring its degree of fluorescence polarization.

Table 5

| | Water | Luminescent particles T1 | Phenol derivative (tyramine hydrochloride) | HRP | Glucose | Target substance GOX (50 μL) | Degree of fluorescence polarization (mp) |
|---|---|---|---|---|---|---|---|
| Stock solution concentration | | 0.08 mg/mL | 40 mM | 2 mg/mL | 100 mg/mL | | |
| | Reagent composition (μL) | | | | | Final concentration of target substance (μg/mL) | |
| Example 5 | 100 | 10 | 30 | 20 | 5 | 0.0 | 62.5 |
| | 100 | 10 | 30 | 20 | 5 | 8.7 | 64.1 |
| | 100 | 10 | 30 | 20 | 5 | 129.8 | 67.8 |
| | 100 | 10 | 30 | 20 | 5 | 259.5 | 72.4 |
| | 100 | 10 | 30 | 20 | 5 | 432.6 | 75.7 |

(Example 6: Fluorescence Polarization Measurement of Glucose)

A MES buffer solution (pH 5.0), the luminescent particle T1 solution, tyramine hydrochloride, HRP and GOX were mixed at the ratios shown in Table 6. The resultant solution was transferred to a 96-well microplate. Next, various concentrations of glucose were each added as a target substance to the solution in the wells, followed by thorough stirring. Then, a degree of fluorescence polarization (mp) was measured in the same manner as in Example 1 except that the degree of fluorescence polarization was measured after 3 minutes.

The results of Example 6 are shown in Table 6. As the concentration of glucose increased, the degree of fluorescence polarization increased. It is conceived that hydrogen peroxide was generated from the target substance glucose by GOX, and the hydrogen peroxide triggered the activation of the peroxidase to aggregate the particles, resulting in the increase in degree of fluorescence polarization.

It was found that by using the relational expression (calibration curve) between the glucose concentration and the degree of fluorescence polarization obtained from this Example, the concentration of glucose having an unknown concentration was able to be determined by measuring its degree of fluorescence polarization.

Table 6

| | MES buffer solution | Luminescent particles T1 | Phenol derivative (tyramine hydrochloride) | HRP | GOX | Target substance glucose (10 μL) | Degree of fluorescence polarization (mp) |
|---|---|---|---|---|---|---|---|
| Stock solution concentration | | 0.08 mg/mL | 40 mM | 2 mg/mL | 1.86 mg/mL | | |
| | Reagent composition (μL) | | | | | Final concentration of target substance (mg/mL) | |
| Example 6 | 100 | 10 | 30 | 20 | 20 | 0.00 | 63.0 |
| | 100 | 10 | 30 | 20 | 20 | 0.21 | 64.4 |
| | 100 | 10 | 30 | 20 | 20 | 0.43 | 67.1 |
| | 100 | 10 | 30 | 20 | 20 | 2.14 | 70.3 |

Various embodiments have been described in detail above but it will be understood that the present disclosure is not limited to these embodiments and encompasses all modifications, variants, alternatives and equivalents falling within the scope of the appended claims.

**Claims**

1. A method of detecting a target substance that is a substance related to an enzyme reaction, the method comprising:

    a first step of mixing a specimen liquid that may comprise the target substance and a reagent to obtain a liquid sample comprising hydrogen peroxide, a peroxidase (40), a phenol derivative and a plurality of luminescent particles (10) each having a binding functional group;
    a second step of aggregating the plurality of luminescent particles (10) through binding of the binding functional groups (20) of the luminescent particles (10) based on a reaction of the hydrogen peroxide, the peroxidase (40) and the phenol derivative in the liquid sample, which occurs when the target substance is present in the liquid sample; and
    a third step of obtaining a value related to fluorescence anisotropy of the liquid sample in the second step.

2. The method of detecting a target substance according to claim 1, wherein the luminescent particles are nanoparticles each comprising a rare earth luminescent complex.

3. The method of detecting a target substance according to claim 1 or 2, wherein the binding functional group is selected from the group consisting of: a thiol group; a carboxy group; an amino group; and a maleimide group.

4. The method of detecting a target substance according to any one of claims 1 to 3, wherein the phenol derivative comprises any one selected from the group consisting of: tyramine; tyramine hydrochloride; phenol; glycyl-L-tyrosine; resorcinol; and serotonin.

5. The method of detecting a target substance according to any one of claims 1 to 4, wherein the target substance is a peroxidase.

6. The method of detecting a target substance according to any one of claims 1 to 4,

    wherein the target substance is an oxidase,
    wherein the liquid sample comprises a substrate for the oxidase, and
    wherein the hydrogen peroxide comprises a product generated by the oxidase.

7. The method of detecting a target substance according to any one of claims 1 to 4, wherein the target substance is hydrogen peroxide.

8. The method of detecting a target substance according to any one of claims 1 to 4,

    wherein the target substance is a substrate for an oxidase,
    wherein the liquid sample comprises the oxidase, and
    wherein the hydrogen peroxide comprises a product generated by the oxidase.

9. The method of detecting a target substance according to any one of claims 1 to 4, wherein the target substance is a phenol derivative.

10. The method of detecting a target substance according to any one of claims 1 to 9, wherein the specimen liquid comprises hydrogen peroxide.

11. A reagent for inspection of a target substance using measurement of a value related to fluorescence anisotropy,

    wherein the target substance is a peroxidase (40), and the reagent comprises hydrogen peroxide, a phenol derivative and a plurality of luminescent particles (10) each having a binding functional group;
    wherein the target substance is a hydrogen peroxide, and the reagent comprises a peroxidase (40), a phenol derivative and a plurality of luminescent particles (10) each having a binding functional group; or
    wherein the target substance is a phenol derivative, and the reagent comprises a peroxidase (40), hydrogen peroxide and a plurality of luminescent particles (10) each having a binding functional group.

12. A reagent for inspection of a target substance using measurement of a value related to fluorescence anisotropy,

wherein the target substance is an oxidase, and the reagent comprises a substrate for the oxidase, a peroxidase (40), hydrogen peroxide, a phenol derivative and a plurality of luminescent particles (10) each having a binding functional group; or

wherein the target substance is a substrate for an oxidase, and the reagent comprises the oxidase, a peroxidase (40), hydrogen peroxide, a phenol derivative and a plurality of luminescent particles (10) each having a binding functional group.

13. The reagent for inspection of a target substance according to claim 11 or 12, wherein the binding functional group is a thiol group.

14. The reagent for inspection of a target substance according to claim 11 or 12, wherein the luminescent particles are particles each comprising a rare earth luminescent complex.

# FIG. 1

```
┌────────────────────────────────────────────────────────────┐
│                        FIRST STEP                          │
│ OF MIXING SPECIMEN LIQUID THAT MAY CONTAIN TARGET SUBSTANCE │ ～S1001
│  AND REAGENT TO OBTAIN LIQUID SAMPLE CONTAINING HYDROGEN    │
│    PEROXIDE, PEROXIDASE, PHENOL DERIVATIVE AND PLURALITY OF │
│   LUMINESCENT PARTICLES EACH HAVING BINDING FUNCTIONAL GROUP│
└────────────────────────────────────────────────────────────┘
                              │
                              ▼
┌────────────────────────────────────────────────────────────┐
│                       SECOND STEP                          │
│  OF AGGREGATING PLURALITY OF LUMINESCENT PARTICLES THROUGH  │
│ BINDING OF BINDING FUNCTIONAL GROUPS OF LUMINESCENT PARTICLES│ ～S1002
│   BASED ON REACTION OF HYDROGEN PEROXIDE, PEROXIDASE AND    │
│    PHENOL DERIVATIVE IN LIQUID SAMPLE, WHICH OCCURS WHEN    │
│      TARGET SUBSTANCE IS PRESENT IN LIQUID SAMPLE          │
└────────────────────────────────────────────────────────────┘
                              │
                              ▼
┌────────────────────────────────────────────────────────────┐
│                        THIRD STEP                          │
│   OF OBTAINING VALUE RELATED TO FLUORESCENCE ANISOTROPY    │ ～S1003
│          OF LIQUID SAMPLE IN SECOND STEP                   │
└────────────────────────────────────────────────────────────┘
                              │
                              ▼
┌────────────────────────────────────────────────────────────┐
│                       FOURTH STEP                          │ ～S1004
│    OF DETECTING TARGET SUBSTANCE BASED ON VALUE           │
│         RELATED TO FLUORESCENCE ANISOTROPY               │
└────────────────────────────────────────────────────────────┘
```

# FIG. 2

**20**

**10**

HIGH MOBILITY

**40**
(TARGET
SUBSTANCE)

PHENOL DERIVATIVE
HYDROGEN PEROXIDE

**40**

**10**

LOW MOBILITY

FIG. 3

GENERATION OF
HYDROGEN PEROXIDE

60

40

40

20

60
(TARGET
SUBSTANCE)

PHENOL DERIVATIVE
GLUCOSE

10

10

HIGH MOBILITY

LOW MOBILITY

# FIG. 4

HIGH MOBILITY

HYDROGEN PEROXIDE (TARGET SUBSTANCE)

PHENOL DERIVATIVE

LOW MOBILITY

# FIG. 5

## EUROPEAN SEARCH REPORT

**Application Number**

EP 25 21 2995

### DOCUMENTS CONSIDERED TO BE RELEVANT

| Category | Citation of document with indication, where appropriate, of relevant passages | Relevant to claim |
|---|---|---|
| X | MUÑOZ-SAN MARTÍN CRISTINA ET AL: "A novel peptide-based electrochemical biosensor for the determination of a metastasis-linked protease in pancreatic cancer cells", ANALYTICAL AND BIOANALYTICAL CHEMISTRY, SPRINGER BERLIN HEIDELBERG, BERLIN/HEIDELBERG, vol. 412, no. 24, 27 January 2020 (2020-01-27), pages 6177-6188, XP037224553, ISSN: 1618-2642, DOI: 10.1007/S00216-020-02418-W [retrieved on 2020-01-27] * page 6180, 2nd para; figure 1 * | 11 |
| A | QING CHANG ET AL: "Sensitive fluorescent probes for determination of hydrogen peroxide and glucose based on enzyme-immobilized magnetite/silica nanoparticles", ANALYTICAL AND BIOANALYTICAL CHEMISTRY, SPRINGER, BERLIN, DE, vol. 395, no. 7, 24 September 2009 (2009-09-24), pages 2377-2385, XP019756670, ISSN: 1618-2650, DOI: 10.1007/S00216-009-3118-9 * abstract; page 2378, right-column, last para - page 2379, left-column, 1st para. * | 1-14 |

-----

-----

-/--

**CLASSIFICATION OF THE APPLICATION (IPC)**

INV.
C12Q1/26
C12Q1/28
G01N33/542
G01N33/58

**TECHNICAL FIELDS SEARCHED (IPC)**

C12Q
G01N

The present search report has been drawn up for all claims

| Place of search | Date of completion of the search | Examiner |
|---|---|---|
| Munich | 9 March 2026 | Lindberg, Pia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches Patentamt

European Patent Office

Office européen des brevets

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ZHAI HONG ET AL: "Development of dual-emission ratiometric probe-based on fluorescent silica nanoparticle and CdTe quantum dots for determination of glucose in beverages and human body fluids", FOOD CHEMISTRY, ELSEVIER LTD, NL, vol. 204, 3 March 2016 (2016-03-03), pages 444-452, XP029446370, ISSN: 0308-8146, DOI: 10.1016/J.FOODCHEM.2016.02.159 * page 447, section 3.2; figure 1 * | 1-14 | |
| A | US 2024/093087 A1 (KAKEGAWA NORISHIGE [JP] ET AL) 21 March 2024 (2024-03-21) * paragraphs [0083]-[0093], [0126]; the claims * | 1-14 | |
| T | DAVID M. JAMESON ET AL: "Fluorescence Polarization/Anisotropy in Diagnostics and Imaging", JOURNAL OF ORGANIC CHEMISTRY, vol. 110, no. 5, 12 May 2010 (2010-05-12), pages 2685-2708, XP055243358, DOI: 10.1021/cr900267p * the whole document * | 1-14 | TECHNICAL FIELDS SEARCHED (IPC) |

The present search report has been drawn up for all claims

| Place of search | Date of completion of the search | Examiner |
|---|---|---|
| Munich | 9 March 2026 | Lindberg, Pia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 21 2995

This annex lists the patent family members relating to the patent documents cited in the above-mentioned European search report.
The members are as contained in the European Patent Office EDP file on
The European Patent Office is in no way liable for these particulars which are merely given for the purpose of information.

09-03-2026

| Patent document cited in search report | Publication date | Patent family member(s) | | Publication date |
|---|---|---|---|---|
| US 2024093087 A1 | 21-03-2024 | CN | 117999308 A | 07-05-2024 |
| | | EP | 4349887 A1 | 10-04-2024 |
| | | JP | 2022187791 A | 20-12-2022 |
| | | US | 2024093087 A1 | 21-03-2024 |
| | | WO | 2022259989 A1 | 15-12-2022 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20080145880
- US 20240093087
- US 20170239356
- WO 2015159995 A